# EUROPÄISCHE PATENTSCHRIFT

(11) **EP 3 801 394 B1**
(45) Veröffentlichungstag und Bekanntmachung des Hinweises auf die Patenterteilung: **31.08.2022**
(21) Anmeldenummer: 19721269.9
(22) Anmeldetag: 30.04.2019
(51) Int. Cl.: A61F 2/28, A61F 2/30

(54) **GELENKIMPLANTAT ZUR GEWEBENEUBILDUNG AM GELENK**
JOINT IMPLANT FOR NEW TISSUE FORMATION AT THE JOINT
IMPLANT ARTICULAIRE POUR LA NÉOFORMATION TISSULAIRE AU NIVEAU DE L'ARTICULATION

(30) Priorität: 11.06.2018 DE 102018113809
(43) Veröffentlichungstag der Anmeldung: 14.04.2021
(73) Patentinhaber: Karl, Christoph, 8274 Tägerwilen (CH)
(72) Erfinder: METHNER, Vilma, 14532 Kleinmachnow (DE); KARL, Christoph, 8274 Tägerwilen (CH)
(74) Vertreter: Kindermann, Peter
(86) Internationale Anmeldenummer: PCT/EP2019/061066
(87) Internationale Veröffentlichungsnummer: WO 2019/238307

(56) Entgegenhaltungen:
- EP-A1- 2 465 549
- US-A- 3 707 006

## Beschreibung

Die vorliegende Erfindung bezieht sich auf ein Gelenkimplantat zur Gewebeneubildung am Gelenk und insbesondere auf ein Gelenkimplantat zur Knorpelneubildung am Knie-, Hüft-, Schulter-, Sprung-, Zehengrund- oder Handgelenk.

Gelenkerkrankungen (Arthrose) gehören zu den zehn häufigsten Erkrankungen weltweit. Arthose ist schmerzhaft und kann ohne Behandlung zur Immobilität der erkrankten Gelenke bis zum totalen Gelenkersatz führen. Ein Totalersatz des erkrankten Gelenks ist hierbei mit hohen Kosten verbunden und wird üblicherweise von den betroffenen Patienten als psychisch belastend empfunden. Revisionen des Gelenkersatzes wirken sich mit weiteren Kosten, Belastungen für den Patienten und häufig Komplikationen aus. Es haben sich daher verschiedene Ansätze zur kurativen Behandlung von Gelenkerkrankungen (Arthrose) und insbesondere zur kurativen Behandlung von Knie- und Hüftgelenks-Arthrose entwickelt, um einen Gelenkersatz zu vermeiden.

Knorpel- und Knochendefekte oder -verluste kann der Säuger nicht ohne weiteres allein reparieren. Spezialisierte Zellen oder sich zu diesen entwickelnde Stammzellen können die Gewebelücken jedoch schließen. Ferner hat Knorpel keinen Zugang zum Blut und dem Knochenmark. Seine Versorgung erfolgt über Diffusion des umliegenden Gewebes. Wenn dieses umliegende Gewebe aber selbst geschädigt ist, erfolgt nur ungenügender Zugang zum regenerationsfähigen Gewebe und den Zellen.

Die derzeit existierenden Ansätze zur kurativen Behandlung von Gelenkerkrankungen werden nachfolgend erläutert.

Medikamentöse Therapieoptionen: Die medikamentösen Therapieoptionen beschränken sich auf die symptomatische Anwendung von Antiphlogistika und Analgetika sowie die teils intraartikuläre und teils systemische Behandlung mit Hyaluronsäure, Chondroitinsulfat, Interleukin-1 Rezeptorantagonisten und Glucosaminsulfat. Obwohl hierbei gute Ergebnisse bei der Schmerzreduktion nachgewiesen wurden, konnte ein Fortschreiten der Arthrose bislang jedoch damit nicht verhindert werden.

Operative Therapien: Es werden gelenkerhaltende Operationen und gelenkersetzende unterschieden. Der Gelenkersatz durch Teil- und Vollprothesen aus Metall und Keramik führt zum Verlust des Gelenkes durch Einsatz von Prothesen. Prothesen werden im Knochen verankert und müssen je nach Lebenszeit ca. alle zehn bis 15 Jahre ersetzt werden. Beim Prothesenwechsel werden häufig die Knochenschäfte vertieft und weiteres Gewebe abgetragen.

Regenerationsmöglichkeiten ergeben sich in unterschiedlicher Art und Weise bei den nachfolgend beschriebenen gelenkerhaltenden Operationen.

Operative Therapien wie lokale Knochen- oder Knorpeltransplantation bzw. autologe Chondrozyten-Transplantation oder - Implantation (ACT oder ACI werden synonym verwendet) konnten sich noch nicht durchsetzen, da hierbei jeweils zwei Operationen notwendig sind (Entnahme und Replantation), was eine lange Entlastung bzw. Ruhigstellung des Gelenks während der Rehabilitation bedeutet. Außerdem wird der noch gesunde Knorpel an der Entnahmestelle geschädigt.

Am weitesten verbreitet sind deshalb operative Therapien wie die sogenannte Pridie-Bohrung, die anterograde/retrograde Anbohrung und die Mikrofrakturierung. Bei diesen operativen Therapien erfolgt kein lokaler Knorpelersatz, sondern es werden z.B. mehrere Bohrungen durch die subchondrale Grenzlamelle durchgeführt. Bei der Bohrung nach Pridie und deren Weiterentwicklung "Mikrofrakturierung" kann im Defektareal ein Einbluten in den Knorpeldefekt erzielt werden, wodurch mesenchymale Stammzellen aus dem spongiösen Raum, Fibroblasten und Chondrozyten in den Knorpeldefekt eingeschwemmt werden. Diese bilden mit Wachstumsfaktoren ein Blutkoagel ("super clot") und differenzieren sich zu artikulärem Knorpel. Klinische Studien zeigten Schmerzverringerung und eine gute Gelenkbeweglichkeit. Ein Problem ist jedoch auch hier eine lange Entlastung bzw. Ruhigstellung des Gelenks, wobei sich ein in der Regel minderwertiger Faserknorpel entwickelt. Dieser ist aufgrund seiner Struktur für die hohen mechanischen Belastungen insbesondere im Kniegelenk häufig nur unzureichend beschaffen und degeneriert schnell, was erneute Eingriffe erforderlich machen kann.

Aus diesem Grund wurden Carbonstifte als Gelenkimplantate zur Gewebeneubildung am Gelenk entwickelt, welche in die Bohrungen eingesetzt werden und zu einem schnellen Überwachsen führen sollen.

Aus der EP 1 450 875 A1 ist ein derartiges Gelenkimplantat zur Gewebeneubildung am Gelenk bekannt, wobei die eingesetzten Stifte aus verdichtetem Kohlenstoff mit einer vorbestimmten Porosität bestehen. Auch bei Verwendung derartiger herkömmlicher Carbonstifte können Fibroblasten und mesenchymale Stammzellen aus dem spongiösen Raum in den Knorpeldefekt eingeschwemmt werden, die einen "super clot" bilden und sich zu artikulärem Knorpel differenzieren.

Das System hat sich jedoch aufgrund zweier wesentlicher Nachteile noch nicht durchgesetzt. Einerseits ist Kohlenstoff als Werkstoff im Knorpel bei Orthopäden nicht akzeptiert, da Mikroabrieb befürchtet wird. Andererseits ist die Oberfläche nicht auf das Ansiedeln von Stammzellen ausgelegt, was sich auch hierbei in der Entwicklung eines minderwertig regenerativen Faserknorpels zeigt.

Aufgrund dieser Limitierungen konnte sich keine der vorstehend genannten Therapien bisher als "Standard of Care" durchsetzen.

Aus der Druckschrift EP 2 465 549 A1 ist ein Implantat in Form einer orthopädischen Befestigungsschraube bekannt, wobei das Implantat einen stiftförmigen Körper mit einem Bodenbereich, einem Deckenbereich und einem Mantelbereich aufweist, und wobei auf dem Mantelbereich des Gelenkimplantats zumindest teilweise eine Gewindestruktur ausgebildet ist. Eine Vor-Form für das Gelenkimplantat kann hierbei mit einem 3D-Druckverfahren ausgebildet werden.

Ferner ist aus der Druckschrift US 3 707 006 A eine Prothese zur Reparatur oder als Ersatz einer Knochenstruktur bekannt, wobei insbesondere eine Zahnprothese mit Gewindestruktur für ein Zahnimplantat offenbart ist.

Der Erfindung liegt demgegenüber die Aufgabe zugrunde, ein Gelenkimplantat zur Gewebeneubildung am Gelenk zu schaffen, welches verbesserte Eigenschaften bei der Gewebeneubildung und der Handhabung aufweist.

Erfindungsgemäß wird diese Aufgabe durch die Merkmale des Patentanspruchs 1 gelöst.

Insbesondere durch die Verwendung eines stiftförmigen Körpers, bei dem zumindest der Deckenbereich des Gelenkimplantats eine hydrophobe Oberfläche zum Zwecke einer chondrozytären Differenzierung und damit der Begünstigung der Chondrogenese von mesenchymalen Stammzellen aufweist, und auf dem Mantelbereich des Gelenkimplantats zumindest teilweise eine Gewindestruktur ausgebildet ist, kann eine Gewebeneubildung und insbesondere eine Knorpelneubildung am Gelenk sowie die Bildung höherwertigen, belastbaren Knorpels verbessert werden. Durch die Verwendung des erfindungsgemäßen Implantats kann eine langanhaltende und durch den fortlaufenden Transport von mesenchymalen Stammzellen in Richtung des Gelenkspaltes nachhaltige Knorpelregeneration erreicht werden, die weitere Eingriffe am Gelenk hinauszögern oder unnötig werden lassen kann. Insbesondere durch die Gewindestruktur wird hierbei ein Platzieren und Entfernen des stiftförmigen Körpers erleichtert und aufgrund der vergrößerten Oberfläche des stiftförmigen Körpers ein verbesserter Transport von Zellen aus dem Knochengewebe an die Gewebeoberfläche (Knorpel) ermöglicht. Darüberhinaus kann mit dem erfindungsgemäßen Gelenkimplantat ein zuverlässiges Fixieren von sog. Scaffolds (Trägermaterialien) und Geweben wie Sehnen und Bänder am Knochen realisiert werden.

Erfindungsgemäß ist der stiftförmige Körper und insbesondere der Deckenbereich und die Gewindestruktur durch eine künstliche Trabekularstruktur mittels eines 3D-Druckverfahrens hergestellt, wodurch der Aufbau sowie die Dimensionierung des stiftförmigen Körpers sehr präzise definiert werden können und der Transport bzw. die Liftfunktion für mesenchymale Stammzellen weiter verbessert ist.

Vorzugsweise stellt der stiftförmige Körper einen Hohlkörper dar, wodurch die Liftfunktion für mesenchymale Stammzellen weiter verbessert ist.

Beispielsweise kann das Material des stiftförmigen Körpers ein nicht-bioresporierbares Polymer, insbesondere PA, PEK, PEKK, PEEK und UHMWPE, oder ein bioresporierbares Polymer, z.B. PCL, ein Metall, insbesondere Ti, insbesondere Reintitan Grade 1, Edelstahl, oder eine Metall-Legierung, insbesondere Ti6Al4V (auch Ti64 genannt), insbesondere CoCr, oder eine nicht-bioresorbierbare Keramik, insbesondere Al2O3, ZrO2 oder eine bioresorbierbare Keramik, insbesondere Ca3(PO4)2, oder eine Kombination von diesen Materialien aufweisen, wodurch ein mechanisch hochfestes Gelenkimplantat mit verbesserten Eigenschaften zur Gebewebeneubildung realisiert werden kann. Vorzugsweise kann das Material des stiftförmigen Körpers Si3N4 aufweisen.

Vorzugsweise kann die hydrophobe Oberfläche durch eine hydrophobe chemische Beschichtung auf einem hydrophilen porösen Untergrund realisiert sein, wodurch sich die chondrozytäre Differenzierung besonders einfach und wirkungsvoll realisieren lässt.

Beispielsweise kann zumindest der Deckenbereich des Gelenkimplantats eine Aussparung zum Einbringen eines Drehmoments aufweisen, wodurch ein besonders präzises Platzieren im Knochen durchgeführt werden kann.

Vorzugsweise kann die Aussparung die Form eines Schlitzes oder Kreuzschlitzes aufweisen, wodurch keine Spezialwerkzeuge für das Platzieren bzw. Entfernen des Implantats notwendig sind. Alternativ kann die Aussparung auch die Form eines Innen-Mehrkants oder Torx aufweisen.

Vorzugsweise kann die Gewindestruktur des Gelenkimplantats eine Gewindesteigung von 0,5 mm bis 1 mm aufweisen, wodurch sich besonders günstige Transporteigenschaften für mesenchymale Stammzellen bei hervorragenden mechanischen Eigenschaften erzielen lassen.

Beispielsweise kann der Bodenbereich des Gelenkimplantats eine Spitze aufweisen und die Gewindestruktur selbstformend, selbstprägend oder selbstschneidend ausgestaltet sein, wodurch das Platzieren des Implantats weiter vereinfacht wird und insbesondere eine Bohrung des Knochens entfallen kann.

Beispielsweise kann die hydrophobe Oberfläche durch eine hydrophobe chemische Beschichtung und insbesondere ein segmentiertes Polymer, vorzugsweise ein Polyurethan oder Polyelektrolyt oder ein hydrophob funktionalisiertes Chitosan oder Chitosanderivat ausgebildet werden. Die Liftfunktion zum Transport von mesenchymalen Stammzellen wird dadurch weiter verbessert.

Alternativ oder zusätzlich kann die hydrophobe Oberfläche durch eine hydrophobe Nanostrukturierung eines Untergrunds realisiert werden, was die chondrozytäre Differenzierung und den Transport von Stammzellen weiter begünstigt.

Vorzugsweise kann der stiftförmige Körper insbesondere im Deckenbereich ferner eine Beschichtung mit einem Wachstumsfaktor, aufweisen, wodurch eine Knorpeldifferenzierung sowie das Anwachsen von Gewebe und insbesondere von Knorpelmaterial am Gelenkimplantat und an der Defektstelle weiter verbessert werden.

Weiterhin kann der stiftförmige Körper insbesondere in seinem Bodenbereich eine hydrophile Oberfläche zur Begünstigung einer osteoblastären Differenzierung von mesenchymalen Stammzellen aufweisen, wodurch eine Knochenbildung und somit eine Verankerung im Knochen verbessert wird.

In den weiteren Unteransprüchen sind weitere vorteilhafte Ausgestaltungen der Erfindung gekennzeichnet.

Die Erfindung wird nachstehend anhand von Ausführungsbeispielen unter Bezugnahme auf die Zeichnung näher beschrieben.

Es zeigen:
Figur 1 eine vereinfachte Schnittansicht eines Oberschenkelknochens mit erfindungsgemäßen Gelenkimplantaten;
Figuren 2A und 2B vereinfachte perspektivische Ansichten von natürlichen Trabekularstrukturen;
Figuren 3A bis 3F vereinfachte perspektivische Ansichten von künstlichen Trabekularstrukturen gemäß Ausführungsbeispielen der Erfindung;
Figur 4 eine vereinfachte perspektivische Ansicht einer Grundstruktur eines Gelenkimplantats gemäß einem ersten Ausführungsbeispiel der Erfindung;
Figur 5 eine vereinfachte perspektivische Ansicht einer Grundstruktur eines Gelenkimplantats gemäß einem weiteren Ausführungsbeispiel der Erfindung;
Figur 6 eine vereinfachte perspektivische Ansicht einer Grundstruktur eines Gelenkimplantats gemäß einem weiteren Ausführungsbeispiel der Erfindung;
Figur 7 eine vereinfachte Seitenansicht eines Gelenkimplantats gemäß einem bevorzugten Ausführungsbeispiel der Erfindung;
Figur 8 eine vereinfachte Teilansicht des Gelenkimplantats gemäß Figur 7;
Figur 9 eine vereinfachte Draufsicht des Gelenkimplantats gemäß Figur 7;
Figur 10 eine vereinfachte Teil-Seitenansicht des Deckenbereichs des Gelenkimplantats gemäß Figur 7;
Figur 11 eine schematische Übersicht der verschiedenen Reifungsstufen von mesenchymalen Stammzellen nach Aubin 1998;
Figuren 12A und 12B ein Ausführungsbeispiel zur Herstellung einer hydrophoben chemischen Beschichtung am Beispiel von segmentierten Polyurethanen.
Figur 13A und 13B vergrößerte Ansichten von mit segmentierten PU beschichteten Ti-Substraten zur Veranschaulichung eines jeweiligen Kontaktwinkels.

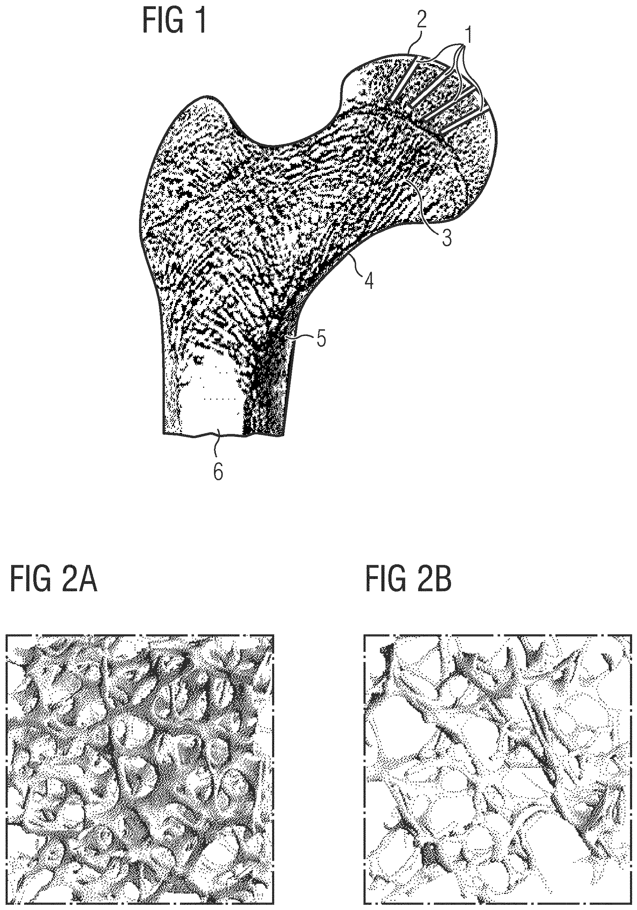

Die Figur 1 zeigt eine vereinfachte Schnittansicht eines oberen Abschnitts eines Oberschenkelknochens zur beispielhaften Veranschaulichung der Verwendung der erfindungsgemäßen Gelenkimplantate zur Gewebeneubildung an einem Oberschenkel-/Hüftgelenk. In Figur 1 sind mit Bezugszeichen 1 die erfindungsgemäßen Gelenkimplantate dargestellt, welche im Bereich des Gelenks in den Oberschenkelknochen eingebracht werden können. Hierbei kann beispielsweise an beschädigten Knorpelbereichen 2 eine oder eine Vielzahl von Vertiefungen in den Knochen gebohrt, gestanzt oder anderweitig ausgebildet und anschließend ein jeweiliges Gelenkimplantat 1 in die jeweils ausgebildete Vertiefung eingebracht werden. Die jeweilige Vertiefung ist hierbei vorzugsweise derart dimensioniert, dass das eingesetzte Gelenkimplantat 1 bzw. dessen Deckenbereich nicht an der Oberfläche des Knochens bzw. Knorpels 2 herausragt, sondern mit dieser eben abschließt oder vorzugsweise der Deckenbereich des Gelenkimplantats 1 unterhalb der Oberfläche des Knochens bzw. Knorpels 2 liegt. Alternativ kann das erfindungsgemäße Gelenkimplantat auch ohne Ausbildung einer Vertiefung direkt in den beschädigten Bereich eingeschraubt werden. Der aufgrund von Gelenkerkrankung (Arthrose) geschädigte Gelenkknorpel 2 kann mit den erfindungsgemäßen Gelenkimplantaten 1 zumindest teilweise regeneriert werden, da an den Enden bzw. Deckenbereichen der eingebrachten Gelenkimplantate 1 eine Neubildung von Gewebe und insbesondere von Gelenkknorpel erfolgt.

Gemäß Figur 1 weist der Knochen eine den Knochen bedeckende Knochenhaut 4 auf, wobei in den Endbereichen 3 des Knochens eine natürliche Trabekularstruktur vorliegt, welche als sogenannte Spongiosa bezeichnet wird. Ferner besteht der Knochen in seinem mittleren Bereich aus einer relativ festen Knochenrinde 5 und in seinem Inneren aus einer Knochenmarkhöhle 6.

Durch die Verwendung einer künstlichen Trabekularstruktur zumindest an einer Oberfläche des Gelenkimplantats 1 können verbesserte Eigenschaften bei der Gewebeneubildung am Gelenk erzielt werden, was nachfolgend im Einzelnen erläutert wird.

Die Figuren 2A und 2B zeigen vereinfachte perspektivische Ansichten von natürlichen Trabekularstrukturen, wie sie beispielsweise im spongiösen Knochenbereich 3 des menschlichen Oberschenkelknochens vorliegen. Hierbei ist gemäß Figur 2A bei einem jungen, gesunden Menschen der spongiöse Knochenbereich 3 mit einer sehr feinen und dichten natürlichen Trabekularstruktur durchsetzt, während gemäß Figur 2B ein älterer und insbesondere ein an z.B. Osteoporose erkrankter Mensch oftmals eine stark veränderte natürliche Trabekularstruktur mit nur wenigen und sehr dünnen Knochenbälkchen (Trabekel) im spongiösen Knochenbereich 3 besitzt.

Erfindungsgemäß kann das Gelenkimplantat 1 einen stiftförmigen Körper aufweisen, der zumindest an seiner Oberfläche oder auch vollständig eine künstliche Trabekularstruktur aufweist. Durch die künstliche und zumindest teilweise offene bzw. für Flüssigkeiten durchlässige Trabekularstruktur des Gelenkimplantats 1 ist beispielsweise ein schnelles Besiedeln der trabekularen Oberfläche und insbesondere des Deckenbereichs des stiftförmigen Körpers mit knorpelbildenden Zellen wie z.B. Chondrozyten ermöglicht, was ein signifikant beschleunigtes und zugleich dauerhaftes Überwachsen zur Folge hat und ferner einen hochwertigen regenerativen Knorpel ermöglicht.

Erfindungsgemäß kann das Gelenkimplantat 1 mit seiner künstlichen Trabekularstruktur durch geeignete 3D-Druckverfahren so aufgebaut und im Hinblick auf die bio-medizinische Anwendung so makro- und mikrostrukturiert werden, dass durch eine definierte Innen- und Außen-Struktur und Rauheit eine optimale Verankerung mit der natürlichen Trabekularstruktur in der Vertiefung (Bohrung, Stanzung etc.) im spongiösen Knochenbereich 3 erfolgt. Eine vertikale, laterale und ggf. rotatorische Beweglichkeit des Gelenkimplantats 1 in der Vertiefung kann dadurch ausgeschlossen werden.

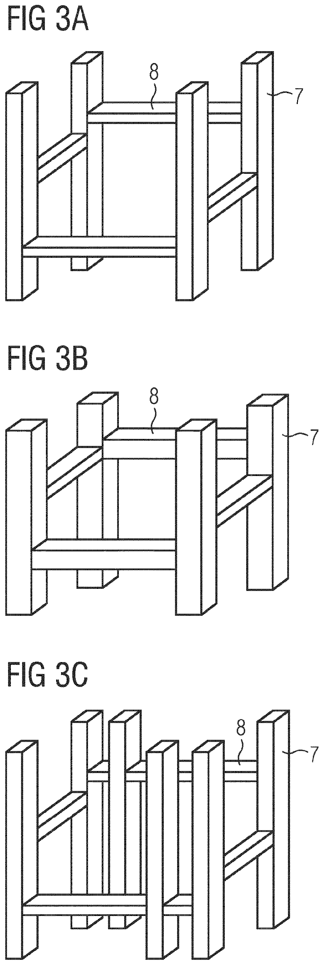

Die Figuren 3A bis 3F zeigen vereinfachte perspektivische Ansichten von künstlichen Trabekularstrukturen gemäß Ausführungsbeispielen der vorliegenden Erfindung. Die künstliche Trabekularstruktur weist hierbei eine Vielzahl von balkenförmigen oder plattenförmigen Elementen (Trabekel) auf, welche miteinander verbunden eine 3-dimensionale Mikroarchitektur ergeben.

Hierbei sollten die durch 3D-Drucktechnik hergestellten, biomimetischen künstlichen Trabekel bestimmte Parameter nicht unterschreiten bzw. überschreiten.

Nachfolgend werden die maßgeblichen Parameter der erfindungsgemäßen künstlichen Trabekularstruktur näher definiert.

Die sogenannte "Mean Trabecular Thickness" (Tb.Th) definiert die durchschnittliche Trabekeldicke der jeweiligen Trabekel bzw. balkenförmigen Elemente. Da beispielsweise gemäß Figur 3A die jeweiligen Trabekel unterschiedlich ausgeformt sein können, stellt Tb.Th den Durchschnitt der lokalen Dicken aller künstlichen Trabekel dar. Die lokale Dicke ergibt sich z.B. bei rechteckförmigen Trabekeln aus der Trabekel-Diagonale und bei kreisförmigen Trabekeln aus dem Trabekel-Durchmesser. Die Figur 3B zeigt schematisch die Auswirkungen auf die künstliche Mikrostruktur bei einer Zunahme der durchschnittlichen Trabekeldicke Tb.Th. Vorzugsweise liegt die durchschnittliche Trabekeldicke Tb.Th für die künstliche Trabekularstruktur in einem Bereich von 100 bis 500 µm und insbesondere von 150 bis 400 µm.

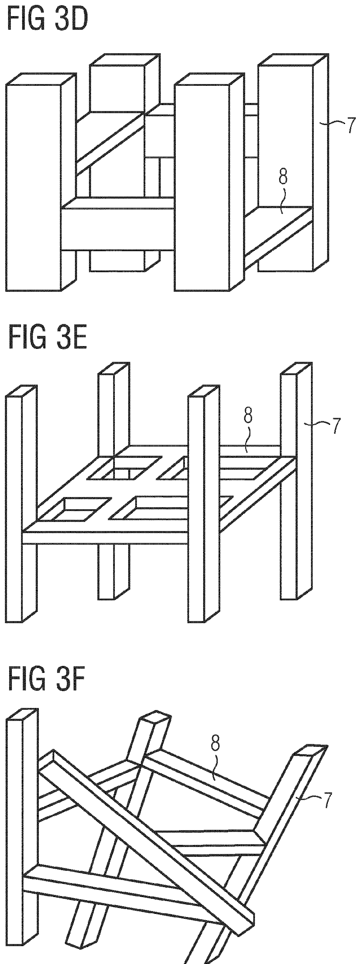

Die sogenannte "Mean Trabecular Separation" (Tb.Sp) definiert den durchschnittlichen Trabekelabstand analog zur durchschnittlichen Trabekeldicke Tb.Th. Eine Abnahme von Tb.Sp kann aus der Änderung verschiedener anderer Parameter resultieren, z.B. Zunahme von Tb.Th (Figur 3B), Zunahme von Tb.N (Figur 3C) oder Abnahme des "Structure Model Index" SMI (Figur 3D). Die Einheit für den durchschnittlichen Trabekelabstand Tb.Sp ist µm und liegt für die erfindungsgemäße künstliche Trabekularstruktur vorzugsweise in einem Bereich zwischen 100 µm und 900µm und insbesondere zwischen 200 µm und 600 µm.

Die sogenannte "Trabecular Number" (Tb.N) ist definiert als Umkehrfunktion der mittleren Distanz zwischen den Achsen der Platten und/oder Balken und gibt die Trabekelanzahl pro mm an. Die Figur 3C zeigt beispielsweise eine Zunahme von Tb.N im Vergleich zu Figur 3A. Vorzugsweise liegt die Trabekelanzahl Tb.N für die künstliche Trabekularstruktur in einem Bereich von 1 bis 6 pro mm, insbesondere in einem Bereich von 1,6 bis 5,2 pro mm.

Der sogenannte "Structure Model Index" (SMI) ist ein weiterer beschreibender Parameter der künstlichen Trabekularstruktur, bei dem es sich beispielsweise um ein aus Platten- und Stabähnlichen Elementen aufgebautes Netzwerk handeln kann. In der Realität nimmt das trabekuläre Netzwerk jedoch nicht die eine oder andere Form an, sondern es besteht ein fließender Übergang. Mit zunehmendem Alter geht z.B. ein eher plattenähnliches Netzwerk in ein eher stabähnliches über. Ausgehend von dieser Erkenntnis wurde daher der sogenannte "Structure Model Index" (SMI) eingeführt, der es ermöglicht, die Struktur in Bezug auf die Anzahl der Platten und Stäbe zu quantifizieren. Für ein ideales Plattenmodell liegt der SMI bei einem Wert von 0 (d.h. reine Plattenstruktur), für ein ideales Stabmodell bei einem Wert von 3. Der SMI beschreibt also die relative Zusammensetzung der künstlichen Trabekularstruktur aus Platten und Stäben. Figur 3D zeigt schematisch eine Abnahme von SMI. Der SMI ist dimensionslos und liegt für die vorliegende Erfindung beispielsweise bei 0,2 bis 2,0, vorzugsweise bei 0,25 bis 1,8.

Die sogenannte "Connectivity-Density (Conn.D) ist ein Maß für die Vernetzung des trabekulären Geflechts. Konnektivität ist die maximale Anzahl von Verbindungen, die innerhalb des Netzwerkes z.B. durch Mikrofrakturen unterbrochen werden können, ohne das Netz als Ganzes in zwei nicht mehr miteinander verbundene Teile zu unterbrechen. Die Figur 3E zeigt schematisch eine Zunahme der Vernetzungsdichte Conn.D. Vorzugsweise liegt die Vernetzungsdichte Conn.D für die erfindungsgemäße künstliche Trabekularstruktur in einem Bereich von 1/mm³ bis 60/mm³, insbesondere von 1,5/mm3 bis 45/mm3.

Beim geometrischen Grad der Anisotropie (DA) handelt es sich um einen Parameter zur Quantifizierung der räumlichen Asymmetrie. Je höher DA, desto mehr liegt eine Orientierung der künstlichen Trabekularstruktur in einer bestimmten Richtung vor. Die Figur 3F zeigt schematisch eine Abnahme von DA. DA ist wie der Parameter SMI dimensionslos. Ein DA von 0 gibt eine perfekt isotrope, ein DA von 1 eine perfekt anisotrope Struktur an. Ergänzend wird der Grad der Anisoptropie auch durch den sogenannten tDA (alternativer DA) mit Werten von 1, perfekt isotrop, bis unendlich, perfekt anisotrop, angegeben. Der tDA findet bei der Beschreibung der erfindungsgemäßen Struktur hier allerdings keine Anwendung. Vorzugsweise liegt der geometrische Grad der Anisotropie DA für die erfindungsgemäße künstliche Trabekularstruktur in einem Bereich von 0,1 bis 1,0, insbesondere von 0,2 bis 0,8 und weiter bevorzugt bei 0,2 bis 0,6.

Beim sogenannten "Bone Volume Tissue Volume Fraction" (BV/TV) handelt es sich um den volumetrischen Anteil der Trabekel am Gesamtvolumen einer betrachteten Trabekularstruktur. Eine Zunahme von BV/TV kann aus der Änderung verschiedener anderer Parameter resultieren, z.B. Zunahme von Tb.Th (Figur 3B), Zunahme von Tb.N (Figur 3C) oder Abnahme von SMI (Figur 3D). Vorzugsweise liegt BV/TV für die erfindungsgemäße Trabekularstruktur in einem Bereich von 6% bis 70%, stärker bevorzugt von 20 bis 50%.

Schließlich definiert das sogenannte "Marrow Star Volume" (MSV) eine jeweilige Trabekel-Porosität der künstlichen Trabekularstruktur. Genauer gesagt bestimmt die MSV die Größe der Hohlräume in der künstlichen Trabekularstruktur. Der arithmetische Mittelwert mMSV liegt erfindungsgemäß vorzugsweise in einem Bereich von 0,05 mm³ bis 110 mm³, insbesondere zwischen 0,05 mm³ bis 9 mm³ und weiter bevorzugt zwischen 0,05 mm³ bis 5 mm³.

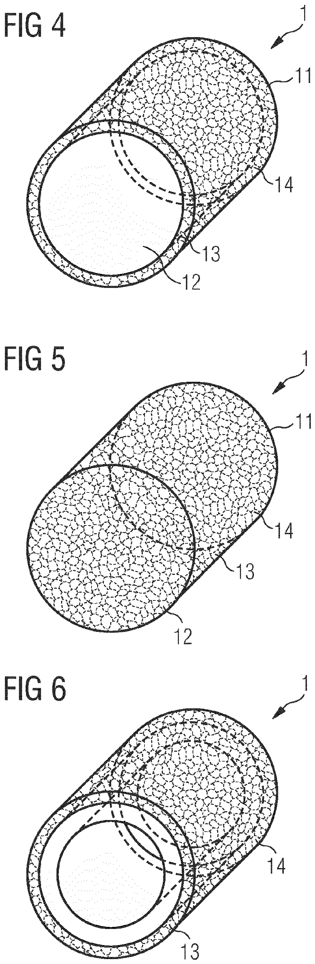

Figur 4 zeigt eine vereinfachte perspektivische Ansicht einer Grundstruktur des Gelenkimplantats 1 gemäß einem ersten Ausführungsbeispiel der Erfindung. Das Gelenkimplantat 1 weist hierbei einen stiftförmigen Körper in Form eines VollZylinders (flüssigkeitsdicht) auf, der einen Bodenbereich 11, einen Mantelbereich 13 und einen Deckenbereich 12 aufweist. Das Gelenkimplantat 1 kann hierbei derart in den Knochen gemäß Figur 1 eingesetzt werden, dass sein Deckenbereich 12 im Bereich des Knorpels 2 vorzugsweise leicht vertieft im Knochen angeordnet ist. Somit kann der Deckenbereich 12 des Gelenkimplantats 1 als Wachstumsbereich für das neu auszubildende Gewebe bzw. den neu auszubildenden Gelenkknorpel 2 wirken. Der Bodenbereich 11 sowie der untere Teil des Gelenkimplantats befinden sich vorzugsweise teilweise oder vollständig im spongiösen Knochenbereich 3.

Gemäß Figur 4 kann zumindest in einem Mantelbereich 13 die vorstehend beschriebene künstliche Trabekularstruktur 14 ausgebildet werden. Ferner kann aber insbesondere auch der Deckenbereich 12 die künstliche Trabekularstruktur 14 aufweisen. Durch die offene und für Körperflüssigkeiten durchlässige Trabekularstruktur des Gelenkimplantats 1 wird beispielsweise ein schnelles Besiedeln der trabekularen Oberfläche mit Zellen wie Chondrozyten ermöglicht, was ein signifikant beschleunigtes Überwachsen zur Folge hat. Ferner ermöglicht das erfindungsgemäße Gelenkimplantat je nach Struktur und Beschichtung das Wachstum eines regenerativen Faserknorpels bis hin zu einem hochwertigen, hyalinen regenerativen Knorpels insbesondere am Deckenbereich 12.

Figur 5 zeigt eine vereinfachte perspektivische Ansicht einer Grundstruktur des stiftförmigen Körpers des Gelenkimplantats 1 gemäß einem weiteren Ausführungsbeispiel der Erfindung, wobei gleiche Bezugszeichen gleiche oder entsprechende Elemente bezeichnen, weshalb nachfolgend auf ihre wiederholte Beschreibung verzichtet wird. Während gemäß Figur 4 der stiftförmige Körper des Gelenkimplantats 1 einen Vollzylinder aufweisen kann, wobei zumindest an dessen Manteloberfläche 13 die erfindungsgemäße Trabekularstruktur 14 ausgebildet ist, kann gemäß Figur 5 der stiftförmige Körper des Gelenkimplantats 1 auch vollständig aus der künstlichen Trabekularstruktur 14 bestehen. Das Einschwemmen, die Proliferation und die Migration von Fibroblasten, mesenchymalen Stammzellen aus dem spongiösen Knochenbereich 3 und Chondrozyten in den Knorpeldefekt kann dadurch weiter verbessert werden.

Figur 6 zeigt eine vereinfachte perspektivische Ansicht einer Grundstruktur des stiftförmigen Körpers des Gelenkimplantats 1 gemäß einem weiteren Ausführungsbeispiel der Erfindung, wobei gleiche Bezugszeichen gleiche oder entsprechende Elemente bezeichnen, weshalb nachfolgend auf ihre wiederholte Beschreibung verzichtet wird. Gemäß Figur 6 kann der stiftförmige Körper des Gelenkimplantats 1 vorzugsweise einen Hohlkörper, wie z.B. einen Hohl-Zylinder, aufweisen, wobei die künstliche Trabekularstruktur 14 zumindest in einem Mantelbereich 13 des Hohl-Zylinders ausgebildet ist. Beispielsweise weist der Höhlkörper zumindest einen zusammenhängenden oder mehrere nicht zusammenhängende Hohlräume auf. Alternativ kann der Hohl-Zylinder aber auch vollständig aus der künstlichen Trabekularstruktur 14 bestehen (nicht dargestellt). Das Einschwemmen, die Proliferation und die Migration von Fibroblasten, mesenchymalen Stammzellen aus dem spongiösen Knochenbereich 3 und Chondrozyten in den Knorpeldefekt kann durch diese Vergrößerung der Interaktionsoberfläche weiter verbessert werden.

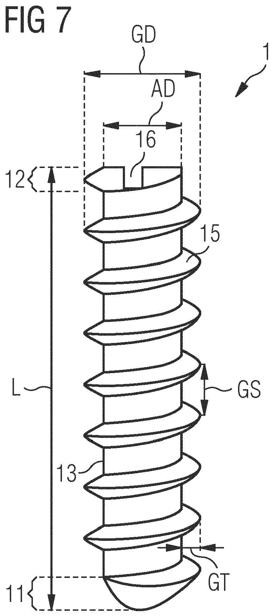

Figur 7 zeigt eine vereinfachte Seitenansicht eines Gelenkimplantats gemäß einem bevorzugten Ausführungsbeispiel der Erfindung, wobei gleiche Bezugszeichen gleiche oder entsprechende Elemente bezeichnen, weshalb nachfolgend auf ihre wiederholte Beschreibung verzichtet wird. Gemäß Figur 7 ist auf dem Mantelbereich 13 des stiftförmigen Körpers zumindest teilweise eine Gewindestruktur 15 ausgebildet. Die Gewindestruktur 15 kann sich durchgehend vom Bodenbereich 11 bis zum Deckenbereich 12 erstrecken oder Unterbrechungen aufweisen. Die Gewindestruktur 15 kann auch nur vom Bodenbereich 11 bis ca. zur Mitte des stiftförmigen Körpers auf dem Mantelbereich 13 ausgebildet sein oder alternativ nur von ca. der Mitte des stiftförmigen Körpers bis zum Deckenbereich auf dem Mantelbereich 13 ausgebildet sein. Der Rest des stiftförmigen Körpers bleibt dann gewindelos.

Die Gewindestruktur 15 kann beispielsweise aus der vorstehend beschriebenen künstlichen Trabekularstruktur ausgebildet sein, sie kann jedoch auch aus einem Vollmaterial ausgebildet sein.

Gemäß Figur 7 kann eine bevorzugte Makrostrukturierung beispielsweise durch ein 3D-Druckverfahren realisiert werden, wobei ein Gewindedurchmesser GD der Gewindestruktur 15 vorzugsweise 3 mm aufweist. Eine Gewindetiefe GT der Gewindestruktur 15 weist vorzugsweise 0,5 mm auf und eine Gewindesteigung GS der Gewindestruktur 15 vorzugsweise im Bereich von 0,5 mm bis 1 mm liegen. Ein Aussendurchmesser AD des stiftförmigen Körpers weist beispielsweise 2 mm auf.

Ferner kann zumindest im Deckenbereich 12 des Gelenkimplantats 1 eine Aussparung 16 zum Einbringen eines Drehmoments ausgebildet sein. Die Aussparung 16 wird durch Makrostrukturierung wiederum vorzugsweise durch ein 3D-Druckverfahren ausgebildet. Beispielsweise wird als Aussparung 16 ein Schlitz zur Aufnahme eines Schraubendrehers bzw. Bits ausgebildet, womit das Gelenkimplantat in den Knochen eingeschraubt werden kann. Ein spezielles Applikationssystem ist somit nicht notwendig, da vorhandene medizinische Schraubendreher verwendet werden können. Die Kosten werden dadurch wesentlich verringert.

Vorzugsweise hat der stiftförmige Körper des Gelenkimplantats 1 eine Länge L von mindestens 0,6 cm und maximal 1,2 cm für die patellare und Applikation in Kleingelenken wie, z.B. dem Hand- oder Sprunggelenk und mindestens 0,8 cm und maximal 2,2 cm, insbesondere 1,0 cm bis 1,6 cm und weiter bevorzugt 1,25 cm, für die jeweils proximale und distale tibiale und femurale Applikation im Knie- und Hüftgelenk. Dadurch kann ein Optimum an Zugänglichkeit und Einwachsen von mesenchymalen Stammzellen ermöglicht werden. Der stiftförmige Körper des Gelenkimplantats 1 kann ferner einen Gewindedurchmesser GD von mindestens 2 mm und maximal 6 mm, vorzugsweise 3 mm, aufweisen, wodurch ein Optimum an lateraler, der Synovia zugewandten Oberfläche zur Ausbildung von Ersatzknorpelgewebe erzielbar ist. Eine Dicke der künstlichen Trabekularstruktur 14 (z.B. des Mantelbereiches 13) beträgt vorzugsweise 0,5 bis 2,0 mm, weiter bevorzugt 0,5 bis 1,5 mm.

Weiterhin kann das Gelenkimplantat 1 gemäß Figur 7 im Bodenbereich 11 eine Spitze oder einen Konus aufweisen und die Gewindestruktur 15 selbstformend, selbstprägend oder selbstschneidend ausgestaltet sein. In diesem Fall kann das Ausbilden einer Vertiefung bzw. einer Bohrung im Knochen entfallen, da sich das Gelenkimplantat 1 selbsttätig in den Knochen eingräbt.

Durch eine definierte, repetitive, an die natürliche Trabekularstruktur angelehnte Netzstruktur der künstlichen Trabekularstruktur oder eine Kanalstruktur mit entsprechend angepasster Form des Gelenkimplantats 1 oder eine Kombination aus Netzstruktur und Kanalstruktur kann ein optimales Einwachsen von körpereigenem Gewebe in das Grenzvolumen zwischen Gelenkimplantat 1 und Vertiefung bzw. Bohrkanal, insbesondere in das Innenvolumen des Mantelbereichs 13 des Gelenkimplantats 1 und oberhalb des zur Synovia (Gelenkspalt) zeigenden Endes des Gelenkimplantats 1 ermöglicht werden.

Durch die Gewindestruktur 15 kann das Gelenkimplantat 1 sehr präzise im Knochen positioniert und hinsichtlich seiner Höhe eingestellt werden. Ferner ergibt sich durch die Gewindestruktur 15 eine verbesserte mechanische Verankerung im Knochen. Darüberhinaus kann das Gelenkimplantat 1 ohne großflächiges Herausfräsen auch wieder entfernt werden. Die Gewindestruktur 15 vergrößert aber insbesondere in vorteilhafterweise die Oberfläche des Gelenkimplantats 1, wodurch ein Transport (Liftfunktion) von Zellen weiter verbessert ist. Durch die spezielle Form der Gewindestruktur 15 können somit noch mehr Zellen aus dem Knochengewebe an die Gewebeoberfläche gelangen, wodurch man eine weiter verbesserte Knorpelausbildung und somit Regeneration erhält.

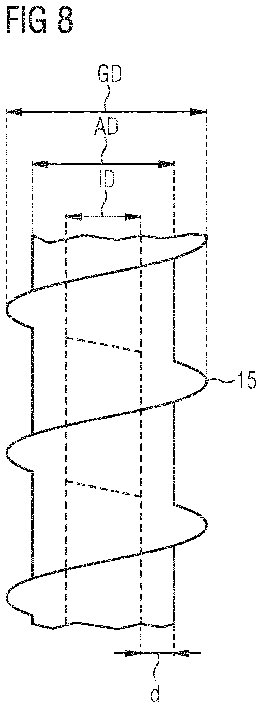

Die Figur 8 zeigt eine vereinfachte Teilansicht des Gelenkimplantats gemäß Figur 7, wobei gleiche Bezugszeichen gleiche oder entsprechende Elemente bezeichnen, weshalb nachfolgend auf ihre wiederholte Beschreibung verzichtet wird. Gemäß Figur 8 kann als stiftförmiger Körper vorzugsweise ein Hohlzylinder verwendet werden, wodurch ein Transport (Liftfunktion) von Zellen aus dem Knochengewebe an die Gewebeoberfläche weiter verbessert wird. Ein Innendurchmesser ID des stiftförmigen Körpers beträgt vorzugsweise 1 mm und eine Dicke d des Mantelbereichs 13 beträgt vorzugsweise 0,5 mm. Beispielsweise können sowohl die Gewindestruktur 15 als auch der Mantelbereich 13 vollständig aus einer künstlichen Trabekularstruktur ausgebildet sein, wodurch sich eine Zell-Liftfunktion weiter verbessert.

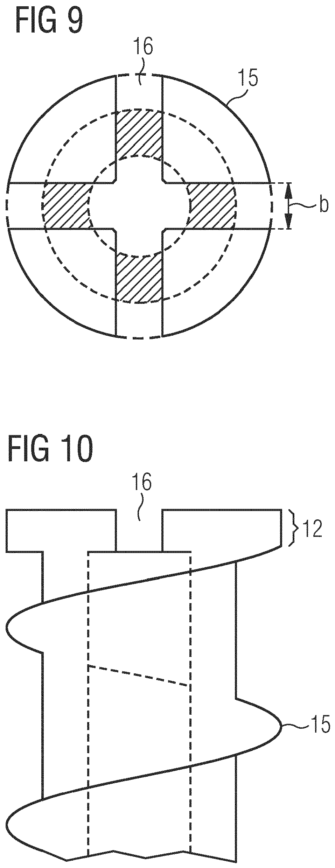

Die Figuren 9 und 10 zeigen eine vereinfachte Draufsicht sowie eine vereinfachte Teil-Seitenansicht des Gelenkimplantats gemäß Figur 7, wobei gleiche Bezugszeichen gleiche oder entsprechende Elemente bezeichnen, weshalb nachfolgend auf ihre wiederholte Beschreibung verzichtet wird. Gemäß Figuren 9 und 10 kann als Aussparung 16 zum Einbringen eines Drehmoments insbesondere ein Kreuzschlitz im Deckenbereich 12 des Gelenkimplantats 1 ausgebildet werden. Eine Schlitzbreite b der Aussparung 16 beträgt beispielsweise 0,5 mm bis 1 mm. Die Schlitze können hierbei über den gesamten Bereich der Hohlzylinderwand und ggf. der Gewindestruktur 15 ausgebildet sein.

Alternativ können zu den vorstehend beschriebenen Ausführungsformen (Schlitz und Kreuzschlitz) auch weitere Formen für die Aussparung 16 realisiert werden (nicht dargestellt). Insbesondere kann die Aussparung 16 die Form eines Innen-Mehrkants (Vierkant, Sechskant usw.) oder eines Torx aufweisen. Bei einer derartigen Ausführungsform kann die Aussparung 16 nicht nur lokal im Deckenbereich 12 sondern als durchgängige Aussparung vom Bodenbereich 11 bis zum Deckenbereich 12 im Hohlraum des stiftförmigen Körpers ausgebildet sein, wodurch die Oberfläche des stiftförmigen Körpers weiter vergrößert wird und eine Liftfunktion für den Transport von Zellen weiter verbessert wird. Zum kontrollierten Setzen der schraubenförmigen Gelenkimplantate 1 können diese beispielsweise über einen vorab eingebrachten Kirschner-Draht eingedreht werden.

Vorzugsweise werden die Gelenkimplantate 1 als mikrostrukturierte Stifte auf Basis von medizinisch zugelassenen, bioinerten und biokompatiblen, 3D-Druck-fähigen Materialien wie beispielsweise nicht-bioresporbierbaren Polymeren, insbesondere Polyamid (PA), Polyetherketone, insbesondere PEK [Polyetherketon], PEKK [Poly(etherketonketon)], PEEK [Polyetheretherketon], Polyethylen (PE), insbesondere UHMWPE [ultra high molecular weight polyethylene], oder z.B. bioresorbierbaren Polymeren, insbesondere PCL [Poly-s-Caprolacton] ausgebildet.

Alternativ können auch, vorzugsweise 3D-Druck geeignete, Metalle und Metall-Legierungen, insbesondere Titan (Reintitan Grade 1), insbesondere Ti64 (Ti6Al4V), Ti64 ELI und TiCP, Edelstahl, insbesondere 316L, und Cobaltchrom-Legierungen, insbesondere CoCr als Materialien für die Gelenkimplantate 1 und insbesondere für deren künstliche Trabekularstrukturen verwendet werden.

Ferner können auch nicht-bioresporbierbare, vorzugsweise 3D-Druck geeignete, Keramiken, insbesondere AluminiumoxidKeramik [Al2O3], und Zirkondioxid-Keramik [ZrO2], oder bioresporbierbare Keramiken, insbesondere Calciumphosphat-Keramik [Ca3(PO4)2] als Materialien für die Gelenkimplantate 1 verwendet werden.

Vorzugsweise kann Si3N4 als Material für das Gelenkimplantat 1 verwendet werden.

Grundsätzlich können auch weitere medizinisch zugelassene, bioinerte und biokompatible, sowie insbesondere 3D-Druckfähige Materialien für die Gelenkimplantate 1 und insbesondere für die künstlichen Trabekularstrukturen 14 gemäß Figuren 3 A bis 3F verwendet werden.

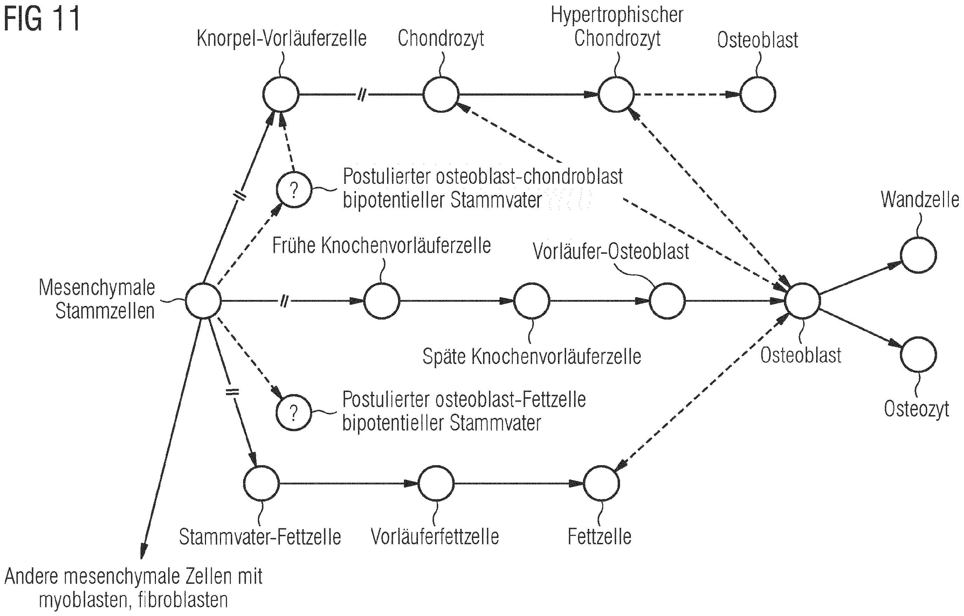

Figur 11 zeigt eine schematische Übersicht der verschiedenen Reifungsstufen von mesenchymalen Stammzellen nach Aubin 1998. Erfindungsgemäß ist es wünschenswert, dass sich mesenchymale Stammzellen (MSC) zumindest im Deckenbereich 12 des stiftförmigen Körpers des Gelenkimplantats 1 zu Chondrozyten differenzieren, um die in diesem Bereich angestrebte Knorpelneubildung zu erzeugen. Andererseits kann im unteren Teil bzw. Bodenbereich 11 des stiftförmigen Körpers des Gelenkimplantats 1 eine Differenzierung der mesenchymalen Stammzellen (MSC) hin zu Osteozyten vorteilhaft sein, um eine Knochenbildung und damit ein optimales Einwachsen des Gelenkimplantats 1 in den spongiösen Knochenbereich 3 zu begünstigen.

Überraschenderweise wurde festgestellt, dass eine derartige Differenzierung von mesenchymalen Stammzellen bereits durch die Erzeugung eines entsprechenden Untergrunds begünstigt werden kann. Genauer gesagt konnte festgestellt werden, dass eine hydrophobe Oberfläche eines Untergrunds eine chondrozytäre Differenzierung von mesenchymalen Stammzellen und somit eine Knorpelbildung begünstigt, während eine hydrophile Oberfläche eines Substrats bzw. Untergrunds eine osteoblastäre Differenzierung von mesenchymalen Stammzellen und somit eine Knochenbildung begünstigt.

Die Begriffe "Hydrophobie" bzw. "hydrophobe Oberfläche" und "Hydrophilie" bzw. "hydrophile Oberfläche" werden nachfolgend über den sogenannten Kontaktwinkel eines Wassertropfens an einer Oberfläche definiert. Hydrophobe Oberflächen weisen hierbei einen Kontaktwinkel größer oder gleich 90° auf, wobei Kontaktwinkel größer 160° superhydrophobe Oberflächen kennzeichnen. Bekanntester Vertreter für diese superhydrophoben Oberflächen ist die sogenannten "Lotospflanze", welche durch ihre besondere Mikro- und Nanostrukturierung Kontaktwinkel von bis zu 170° aufweist. Andererseits sind hydrophile Oberflächen durch einen Kontaktwinkel kleiner 90° gekennzeichnet.

Erfindungsgemäß wird nunmehr diese Differenzierungs-Eigenschaft der Stammzellen in Abhängigkeit von der Hydrophobie oder Hydrophilie einer Oberfläche dahingehend ausgenutzt, dass der stiftförmige Körper des Gelenkimplantats 1 entsprechende hydrophobe Oberflächen aufweist, die eine chondrozytäre Differenzierung der mesenchymalen Stammzellen und somit eine Knorpelausbildung begünstigen.

Hierbei kann sowohl der gesamte stiftförmige Körper eine hydrophobe Oberfläche aufweisen oder aber nur ein Teil des Körpers hydrophobe Oberflächen besitzen. Beispielsweise weist zumindest der Deckenbereich 12 eine hydrophobe Oberfläche auf, um ein Knorpelwachstum an dieser Stelle zu begünstigen. Andererseits kann der stiftförmige Körper in seinem Deckenbereich 12, oberen Mantelbereich 13 und oberen Gewindebereich 15 eine hydrophobe Oberfläche aufweisen, während der Bodenbereich 11, der untere Teil des Mantelbereichs 13 und der untere Teil des Gewindebereiches 15 eine hydrophile Oberfläche aufweist. Dadurch kann im oberen (aus dem Knochen herausragenden) Bereich des Gelenkimplantats 1 ein Knorpelwachstum und im unteren (im Knochen befindlichen) Bereich des Gelenkimplantats ein Knochenwachstum begünstigt werden.

Erfindungsgemäß kann die hydrophobe und damit eine die chondrozytäre Differenzierung begünstigende Oberfläche auf verschiedene Art und Weise realisiert werden. Einerseits können chemische Beschichtungen auf den stiftförmigen Körper und insbesondere dessen Trabekelstrukturen aufgebracht werden, welche die hydrophoben (wasserabweisenden) Eigenschaften verbessern. Andererseits können auch geeignete Mikro- und/oder Nanostrukturierungen der Oberfläche des stiftförmigen Körpers und insbesondere dessen Trabekelstrukturen eine gewünschte hydrophobe Eigenschaft hervorrufen. Weiterhin können auch Kombinationen derartiger hydrophober chemischer Beschichtungen mit physikalischen Topographie-Strukturierungen (Mikro-und/oder Nanostrukturierung) zur Realisierung derartiger "Lo-toseffekt"-Oberflächen mit hydrophoben Eigenschaften verwendet werden, um eine chondrozytäre Differenzierung und damit ein Knorpelwachstum zu begünstigen.

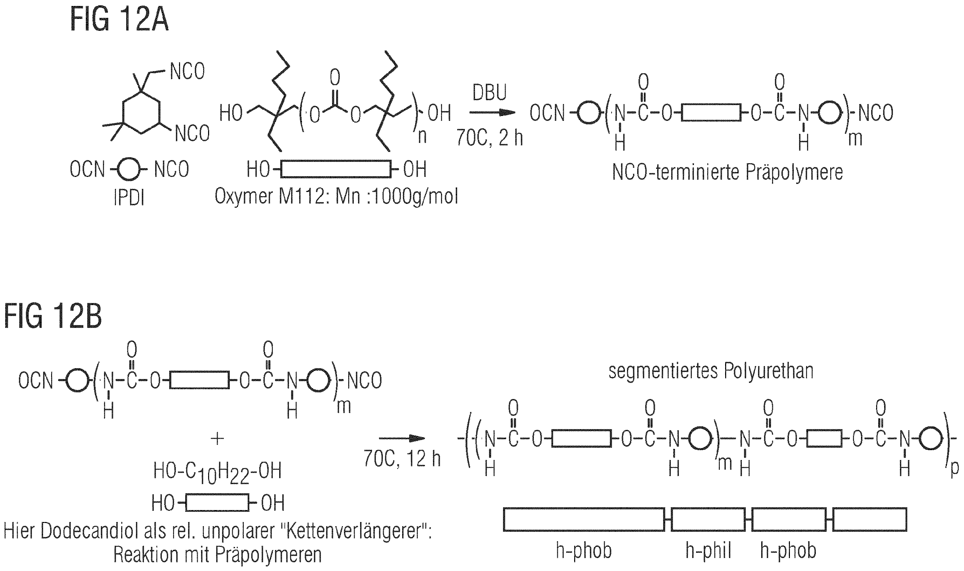

Figuren 12A und 12B zeigen ein Ausführungsbeispiel zur Herstellung einer derartigen hydrophoben Beschichtung am Beispiel von segmentierten Polyurethanen, wie sie auf ein erfindungsgemäßes Gelenkimplantat aufgebracht werden kann.

Gemäß Figur 12A wird zunächst die Herstellung von NCO-terminierten Präpolymeren veranschaulicht, wobei ein stöchiometrischer Überschuss von -NCO vorliegt. Gemäß Figur 12B werden anschließend die NCO-terminierten Präpolymere unter Verwendung von Dodecandiol als unpolaren "Kettenverlängerer" in das gewünschte segmentierte Polyurethan (segmentiertes PU) umgewandelt.

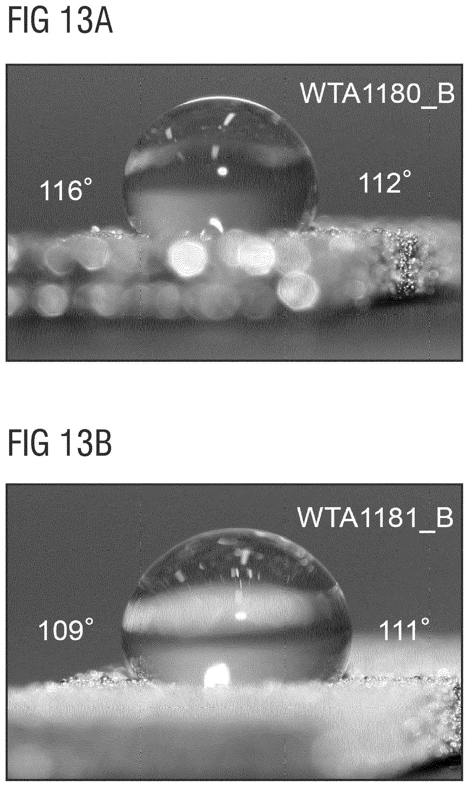

Figur 13A zeigt eine vergrößerte Ansicht eines mit einem derartigen segmentierten PU beschichteten Ti-Substrats. Während ein unbeschichtetes Ti-Substrat (nicht dargestellt) einen Kontaktwinkel von 0° aufweist, besitzt die mit segmentiertem PU beschichtete (10% PU in Toluol) Ti-Oberfläche einen Kontaktwinkel von ca. 112° bis 116°.

Figur 13B zeigt eine vergrößerte Ansicht eines mit einem segmentierten PU beschichteten Ti-Substrats, wobei eine Konzentration des segmentierten PUs bei 2% in Toluol liegt. Die mit einer derartigen segmentierten PU beschichtete Ti-Oberfläche weist nunmehr einen Kontaktwinkel von ca. 109° bis 111° auf.

Für die vorstehend beschriebene hydrophobe PU-Beschichtung wurden folgende Komponenten verwendet:
a) Aliphatische Diisocyanate: Isophoron-Diisocyanat (IPDI), Hexamethylendiisocyanat (HDI) und Dicyclohexylmethandiisocyanat (Hydriertes MDI, HMDI)
b) Polyole: Polycarbonat-Diole (Hydrolysebeständigkeit), wie z.B. Desmophen C2200, Desmophen XP2586, sowie Kohlenwasserstoff-Diole auf der Basis von Naturkautschuk und hydriertem Naturkautschuk
c) Kettenverlängerer: aliphatische Diole wie Hexandiol, Decandiol und eventiuell längere Diole wegen der Hydrophobie

Ferner kann als hydrophobe chemische Beschichtung auch ein Polyelektrolyt-Komplex auf PU-Basis verwendet werden. Hierbei werden die gleichen Komponenten wie oben verwendet, wobei zusätzlich sulfonierte Diole oder ammoniumgruppenhaltige Diole als Kettenverlängerer verwendet werden, um ionische Gruppen für die Bildung der Elektrolytkomplexe einzuführen.

Die Komplexbildung erfolgt dann nach Beschichtung (vorzugsweise Tauchbeschichtung) durch Eintauchen in eine verdünnte Lösung mit einem Tensid (kationisch oder anionisch, je nach dem, welche ionischen Gruppen im Polymer vorhanden sind). Die ionischen Wechselwirkungen zwischen dem Polyelektrolyten und dem Tensid führen zu einer festen Bindung, vor allem, wenn das Tensid hydrophob ist und daher ohnehin in wässriger Umgebung keine Tendenz hat, in Lösung zu gehen.

Ferner kann als hydrophobe chemische Beschichtung auch ein Polyelektrolyt-Komplex auf Acrylat-Basis verwendet werden, wobei eine erste Schicht aus Polyelektrolyten wie Polyacrylsäure bzw. acryl- oder methacrylsäurehaltigen Copolymeren, evtl. auch mit einigen Phosphorsäuregruppen (Comonomer Vinylphosphonsäure) zur Haftung auf der Oberfläche aufgebracht wird und anschließend eine zweite Schicht wie oben aufgebracht wird (Beschichtung aus einer Tensid-Lösung, abgestimmt auf die ionischen Gruppen des Polyelektrolyten).

Vorzugsweise werden folgende drei Typen von hydrophoben Beschichtungsmaterialien verwendet:
Polyurethan vernetzt,
Polyurethan unvernetzt,
Polyelektrolyt-Komplexe,
welche nachfolgende Charakteristika aufweisen:
   Polyurethan vernetzt:
   Polyurethane unterscheiden sich von den meisten anderen Polymeren und Kunststoffen dadurch, daß sie durch ein "Baukastensystem" aus vielen unterschiedlichen Komponenten (Diisocyanaten, Polyisocyanaten, Polyolen, Kettenverlängerern, Weichsegmenten etc.) zusammengesetzt werden. Dabei erfolgt der eigentliche Aufbau (chemische Synthese der Polymermoleküle) typischerweise erst während der Verarbeitung, so daß der Anwender bzw. Hersteller von Bauteilen auf der Basis von Polyurethanen die endgültigen Eigenschaften ganz nach seinen Anforderungen zusammenstellen kann. Nahezu alle anderen Kunststoffe werden dagegen mit festen Eigenschaftsprofilen vom Rohstoffhersteller (Chemische Industrie) hergestellt und ausgeliefert, so daß der Anwender bzw. Hersteller von Bauteilen nur verhältnismäßig geringen Einfluß auf das Eigenschaftsprofil hat. Daher stellen Polyurethane eine sehr gute Ausgangsbasis für Spezialentwicklungen wie der Beschichtung des erfindungsgemäßen Implantatkörpers 1 dar.

Polyurethane werden seit langem als biokompatible Werkstoffe eingesetzt, beispielsweise als inerte, nicht-abbaubare Beschichtung von Herzschrittmachern, oder auch als biokompatible, abbaubare Trägermaterialien (Scaffolds) für Tissue Engineering bzw. Regenerative Medizin. Die Eigenschaften (z.B. Hydrophobie/Hydrophilie, Abbaubarkeit/Langzeitstabilität, Festigkeit, Steifigkeit, Porosität etc.) werden dabei durch die Kombination der Komponenten nach Bedarf eingestellt. Vernetzte Polyurethane werden in verdünnter Lösung in Gegenwart des zu beschichtenden Substrats hergestellt. Dabei können die Komponenten so gewählt werden, daß während der Vernetzung gleichzeitig auch eine chemische Anbindung an die Oberfläche des zu beschichtenden Substrats erfolgt. Diese Materialien haften oft ohne Haftvermittler oder ähnliche Zwischenschichten hervorragend, besonders auf hydrophilen Oberflächen. Dabei können die Komponenten der Polyurethane so gewählt werden, daß die entstehenden Schichten selbst hydrophob sind.

Als Komponenten sind für die Biokompatibilität aliphatische Di- und Polyisocyanate geeignet, für die Langzeitstabilität Weichsegmente und Polyole auf Polycarbonat-, Silicon- oder Polybutadien-Basis, und für die Hydrophobie langekttige Diole, eventuell ebenfalls auf Silicon- oder Polybutadien-Basis als Kettenverlängerer.

Der Hauptnachteil ist die problematische Kontrolle der Schichtdicke bei der Beschichtung. Die Konzentration ist der einzige unabhängige Parameter, dessen Variation die Schichtdicke beeinflussen kann. Zwar wirkt sich auch die Zusammensetzung und die Reaktionszeit auf die Schichtdicke aus, allerdings beeinflusst die Zusammensetzung auch alle anderen Eigenschaften, und die Reaktionszeit kann nicht beliebig festgesetzt werden, denn für die Biokompatibilität ist die vollständige Umsetzung der Isocyanatgruppen erforderlich, so daß die Beschichtungszeit nicht beliebig verkürzt werden kann.

Polyurethane, unvernetzt:
Unvernetzte Polyurethane werden getrennt vom Beschichtungsvorgang hergestellt und anschließend aus einer verdünnten Lösung in einem Tauchvorgang aufgebracht. Die Einstellung der Eigenschaften bietet die gleichen Möglichkeiten wie bei den vernetzten Polyurethanen, da fast alle Komponenten in beiden Fällen eingesetzt werden können.

Der Vorteil der unvernetzten Polyurethane liegt darin, daß Synthese und Beschichtung getrennt voneinander ablaufen, so daß bessere Möglichkeiten zur Steuerung der Schichtdicke bestehen. Die Konzentration, die Einwirkzeit beim Tauchvorgang, und vor allem die Anzahl der Tauchvorgänge (mit jeweils Trocknung dazwischen) bestimmen die Dicke der aufgebrachten Schicht.

Der Nachteil liegt darin, daß eine chemische Anbindung der Schicht an das Substrat entweder eine Haftvermittler-Schicht erfordert, oder spezielle Komponenten im Polyurethan, die mit der Oberfläche reagieren können. Unter Umständen ist die Haftung dieser Schichten daher weniger dauerhaft, oder der Beschichtungsvorgang ist aufwändiger, da vor der eigentlichen Beschichtung zunächst eine Haftvermittlerschicht aufgebracht werden muß. Da dies aber voraussichtlich ebenfalls als einfache Tauchbeschichtung möglich ist, ist der zusätzliche Aufwand begrenzt.

Polyelektrolytkomplexe:
Polyelektrolytkomplexe machen sich elektrostatische Wechselwirkungen zwischen positiv und negativ geladenen Ionen und Oberflächen zu nutze. Jedes Material weist in Wasser eine bestimmte Oberflächenladung auf (Zeta-Potential), die - je nach der chemischen Struktur - positiv oder negativ ist. Diese Oberflächenladung besitzen auch neutrale Partikel bzw. Oberflächen. Polyelektrolyte, deren Ladungen entlang der Polymerkette dieser Oberflächenladung entgegengesetzt geladen sind, haften auf der Oberfläche sehr fest. Im Allgemeinen können sie nicht mehr entfernt werden, da jede Polymerkette je nach Kettenlänge mit dutzenden oder hunderten Gruppen gleichzeitig haftet und so selbst dann in Position gehalten wird, wenn einige dieser Gruppen durch äußere Einwirkungen abgelöst werden. Auf diese Polyelektrolyte können dann entweder gegensinnig geladene Polyelektrolyte abgeschieden werden, so daß eine auf molekularer Basis genaue Einstellung der Schichtdicke durch abwechselnde Anscheidung (layer-by-layer-Technik) möglich ist. Oder es werden niedermolekulare Ionen, z.B. Tenside bzw. Seifen abgeschieden, deren eines Ende eine dem Polyelektrolyten gegensinnige Ladung trägt um die Haftung sicherzustellen, und deren anderes Ende hydrophob ist. Im Idealfall können so Schichten hergestellt werden, die nach Außen hin eine dichte Schicht an z.B. Methylgruppen aufweisen, womit nahezu die Oberflächenspannung von Fluorpolymeren (PTFE, Teflon) erreicht werden kann.

Die Vorteile dieser Materialien liegen in der üblicherweise hervorragenden Haftung in wässrigen oder nicht-wässrigen Systemen, in der exakten Steuerbarkeit der Schichtdicke, und in der relativ gut kontrollierbaren, sehr ausgeprägten Hydrophobie.

Nachteilig ist die Abscheidung in nahezu monomolekularen Schichten, die bei größeren Schichtdicken ein große Zahl von Tauchvorgängen in abwechselnden Polyelektrolytbädern erfordert. Da allerdings keine Trockenschritte dazwischen erforderlich sind, ist der Aufwand vertretbar.

Ferner können als Ausgangsmaterial für den stiftförmigen Körper des Gelenkimplantats 1 derartige 3D-druckfähige Materialien verwendet werden, die bereits per se (also ohne zusätzliche Mikro- und/oder Nanostrukturierung und/oder chemische Beschichtung eine hydrophobe Oberfläche aufweisen. Beispielsweise zeigt die unbehandelte Oberfläche eines Zirkondioxid-Keramik-Substrats bereits hydrophobe Eigenschaften.

Alternativ oder zusätzlich zur vorstehend beschriebenen hydrophoben chemischen Beschichtung oder Materialauswahl können ferner mikro- und nanostrukturierte Oberflächen am stiftförmigen Körper des Gelenkimplantats 1 erzeugt werden, wie sie als selbstreinigende Oberflächen auch unter dem Begriff Lotus-Effect^{®} bekannt sind. Derartige mikro- und nanostrukturierte Oberflächen sind beispielsweise aus der Druckschrift WO 96/04123 A bekannt.

Darüber hinaus können die künstlichen Trabekularstrukturen 14 zum Zweck der verbesserten Knorpelzelldifferenzierung und des verbesserten Anwachsens von Knorpelmaterial eine zusätzliche Wachstumsbeschichtung bzw. einen Wachstumsfaktor aufweisen. Vorzugsweise kann die künstliche Trabekularstruktur 14 mit spezifischem, humanem und human-homologem Wachstumsfaktor FGF [Fibroblast Growth Factor] insbesondere FGF-1, FGF-2 und FGF-10 bis FGF-22 und dort insbesondere FGF-18 beschichtet werden. Alternativ kann die künstliche Trabekularstruktur 14 mit spezifischem, humanem und human-homologem Wachstumsfaktor SDF [Stromal Cell-Derived Factor] insbesondere SDF-1 beschichtet werden. Ferner kann spezifischer, humaner und human-homologer Wachstumsfaktor IGF-1 [Insulin-like Growth Factor 1], humanes PDGF [Platelet-Derived Growth Factor], spezifischer, humaner und human-homologer Wachstumsfaktor TGF-β1 und TGF-β3 [Transforming Growth Factors beta 1 und beta 3], oder spezifischer, humaner und human-homologer BMP-2 und BMP-7 [Bone Morphogenetic Protein-2 und Protein-7] auf die künstliche Trabekularstruktur 14 aufgebracht werden. Weitere Möglichkeiten der Beschichtung umfassen: spezifisches, humanes und humanhomologes OP-1 [Osteogenic Protein-1], humanes PRP [Plateletrich Plasma]sowie speziell für Beschichtungen geeignetes bio-inertes Polyamid. Selbstverständlich sind auch Kombinationen der vorstehend beschriebenen Beschichtungen möglich. Vorzugsweise kann der Wachstumsfaktor als letzte Schicht aufgebracht werden.

Erfindungsgemäß kann durch entsprechende Auswahl von geeigneten bioinerten und biokompatiblen Materialien mit idealer Anpassung der geometrischen und chemisch/biochemischen Oberflächenstruktur (künstliche Trabekularstruktur) die Differenzierung von mesenchymalen Stammzellen zu Chondrozyten oder Osteoblasten gezielt gesteuert werden. Dadurch kann insbesondere die Knorpelstruktur auf der der Synovia zugewandten Seite (Deckenbereich 12) der Gelenkimplantate 1 durch die vorstehend beschriebenen hydrophoben Oberflächenstrukturen und Beschichtungen sowie mit den die Knorpelbildung anregenden Wachstumsfaktoren verbessert werden. Weiterhin kann auf der der Synovia abgewandten Seite (Bodenbereich 11) der Gelenkimplantate durch hydrophile Oberflächenstrukturen und Beschichtungen die Knochenbildung sowie die Knochenstruktur im spongiösen Knochenbereich verbessert werden. Dadurch erhält man nahezu physiologische Adäquatheit, da die Gelenkanatomie und die natürliche Knochenstabilität nicht oder nur unbedeutend beeinflusst werden wie z.B. bei Implantation einer Endoprothese. Die Verträglichkeit und Wirksamkeit der kurativen Therapie mittels vorstehend beschriebener Gelenkimplantate ist dadurch wesentlich verbessert.

Das erfindungsgemäße Gelenkimplantat ermöglicht durch seine zylindrische Form mit Außengewinde und unterstützt durch seine hydrophobe Oberfläche, dass Zellen aus dem Knochenmark an dem Stift aufsteigen können, um das um und über dem Stift befindende Gewebe zu erreichen. Der Stiftkörper kann dabei hohl oder gefüllt sein. Das Gelenkimplantat wird in den Knochen geschraubt und versorgt Knochen und das darüber liegende Knorpelgewebe. Das Gelenkimplantat ermöglicht insbesondere den Transport von Zellen, auch wenn kein Anschlußgewebe vorhanden ist. Dadurch können neue Gewebenester entstehen, wie z.B. bei einer ausgedehnten Arthrose oder der entzündliche Knochen kann, z.B. bei Ausbildung eines Ödems über den Zugang zu Zellen den Knochengewebes ausheilen.

Da das Gelenkimplantat im platzierten Knochen bzw. Gewebe verbleibt, kann der Zelltransport fortlaufend erfolgen. Dies ermöglicht erstmals eine quasi-kurative Behandlung.

Das erfindungsgemäße Gelenkimplantat ermöglicht einen Transport von Zellen aus dem Knochengewebe an die Gewebeoberfläche (sog. Liftfunktion). Durch verschiedene Strukturfaktoren kann die Oberfläche des Stiftes stark vergrößert werden und es gelangen mehr Zellen an die Oberfläche (Deckenbereich). Die Strukturfaktoren umfassen beispielsweise eine Makrostrukturierung, wie z.B. eine Kanalbildung im Inneren des Implantatkörpers oder z.B. die Grundform der Gewindestruktur, und eine Mikro- bis Nanostrukturierung wie z.B. die trabekuläre aber auch andersartige, z.B. kanalartige Mikrostrukturierung an der Aussen- und evtl. Innenseite des stiftförmigen Körpers. Die Makro- und Mikro- bis Nanostrukturierung erfolgt vorzugsweise durch geeignete 3D-Druckverfahren und evtl. anschliessende chemische Behandlung der Oberflächen. Dadurch wird die Oberfläche des Stiftes stark vergrößert und mehr Zellen gelangen an die Oberfläche. Der Stift soll vorzugsweise mit der Gewebeoberfläche des Knochens abschließen. Alternativ kann der Stift leicht darunter oder leicht darüber abschließen.

An der Gewebeoberfläche/-grenze können dadurch die Zellen aus dem Knochen und Knochenmark neues fehlendes Gewebe bilden.

Das erfindungsgemäße Gelenkimplantat kann darüber hinaus auch zum Fixieren von Stützstrukturen (sog. Scaffolds) und Geweben wie Sehnen und Bänder am Knochen verwendet werden. Ebenso kann er in der plastischen Chirurgie Verwendung finden, dabei dient es als Träger von Zellen und Verbinder von Geweben oder Geweben und Medizinprodukten und Materialien aber auch zur Bildung von kollagenem Stützgewebe (Callusbildung) z.B. in der Gesichtschirurgie verwendet werden.

Das erfindungsgemäße Gelenkimplantat kann darüber hinaus auch unterbrochene Knochengewebsschichten verbinden, wie z.B. bei Knochenbruch. Hierbei kann durch geeignete Marko-, Mikro- und Nanostrukturierung der Oberflächen und/oder geeignete chemische Nachbehandlung und/oder Beschichtung die Hydrophobie, auch innerhalb des Stiftes alternierend, so eingestellt (adjustiert) werden, dass jeweils eine optimale Besiedlung mit Knochen- oder Knorpelzellen erzielt wird.

Darüber hinaus beschränkt das Gelenkimplantat keine Gabe von Antiresorptiva wie Anti-RANK-L Antikörpern (Denosumab) oder Cathepsin K-Inhibitoren bzw. -Antikörper (Odanacatib) oder Bisphosphonaten, insbesondere Bisphosphonaten und insbesondere parenteralen Bisphosphonaten wie Ibandronsäure und Zoledronsäure und Pamidronsäure und knochenstärkenden (knochenanabolen) Arzneimitteln. Es ermöglicht neben der Kombination mit Antiresorptiva und/oder Knochenanabolika, wie z.B. Teriparatid ("Forsteo^{®}", PTH 1-84 ("Preotact^{®}")oder Abaloparatid ("Tymlos") oder Anti-Sklerostin-Antikörpern (Romosozumab, AMG 785)) die Kombination mit z.B. Hyaluronsäure aber auch anderen Dämpferflüssigkeiten oder Gelen als Synoviaersatz.

Durch die Kombination der vorstehend beschriebenen biokompatiblen, bio-inerten, 3D-druckfähigen Materialien, der spezifisch geeigneten bio-medizinischen Geometrien (Krümmung, Nanostruktur, Mikrostruktur und Makrostruktur) und der wachstumsfördernden Beschichtungen erhält man ein neuartiges Gelenkimplantat, welches die Quantität und Qualität und damit die Belastbarkeit und Beständigkeit von Ersatzknorpelgewebe weiter optimieren kann und wesentlich zur kurativen Behandlung von Gelenkerkrankungen (Arthrose) beitragen kann.

Die Erfindung wurde vorstehend anhand von bevorzugten Ausführungsbeispielen beschrieben. Sie ist jedoch nicht darauf beschränkt und umfasst insbesondere auch Einzel-Kombinationen der vorstehend beschriebenen Ausführungsbeispiele, wenn sie im Umfang der vorliegenden Ansprüche sind. Insbesondere kann auch Kohlenstoff als Material für die Gelenkimplantate und insbesondere für die künstlichen Trabekularstrukturen verwendet werden. Insbesondere kann anstelle der beschriebenen Zylinderform auch eine leicht konische Form für den stiftförmigen Körper des Gelenkimplantats verwendet werden. Insbesondere kann auch die Vorstufe der chondrozytären Differenzierung von mesenchymalen Stammzellen, nämlich die chondroblastäre Differenzierung von mesenchymalen Stammzellen, durch die hydrophobe Oberfläche begünstigt werden.

Obwohl die Erfindung vorstehend im Zusammenhang mit der Verwendung in menschlichen Hüft- und Kniegelenken beschrieben wurde, ist sie nicht darauf beschränkt und umfasst insbesondere auch menschliche Klein- sowie Kleinstgelenke (z.B. Fußsowie Finger-Gelenke) und Gelenke von Tieren.

### Bezugszeichenliste

- 1: Gelenkimplantat
- 2: Gelenkknorpel
- 3: spongiöser Knochenbereich
- 4: Knochenhaut
- 5: Knochenrinde
- 6: Knochenmarkhöhle
- 7, 8: Trabekel
- 11: Bodenbereich
- 12: Deckenbereich
- 13: Mantelbereich
- 14: künstliche Trabekularstruktur
- 15: Gewindestruktur
- 16: Aussparung
- GD: Gewindedurchmesser
- GS: Gewindesteigung
- AD: Aussendurchmesser
- ID: Innendurchmesser
- L: Länge des Gelenkimplantats
- d: Dicke des Mantelbereiches
- b: Breite der Aussparung

## Patentansprüche

1. Gelenkimplantat zur Gewebeneubildung an einem Gelenk, wobei das Gelenkimplantat (1) einen stiftförmigen Körper mit einem Bodenbereich (11), einem Deckenbereich (12) und einem Mantelbereich (13) aufweist,
wobei auf dem Mantelbereich (13) des Gelenkimplantats (1) zumindest teilweise eine Gewindestruktur (15) ausgebildet ist, und
**dadurch gekennzeichnet, dass**
zumindest der Deckenbereich (12) und die Gewindestruktur (15), insbesondere der gesamte stiftförmige Körper, des Gelenkimplantats (1) eine künstliche Trabekularstruktur (14) aufweisen, welche durch ein 3D-Druckverfahren derart hergestellt ist, dass eine hydrophobe Oberfläche zur Begünstigung einer chondrozytären Differenzierung von mesenchymalen Stammzellen realisiert wird.

2. Gelenkimplantat nach Patentanspruch 1, **dadurch gekennzeichnet, dass** der stiftförmige Körper des Gelenkimplantats (1) einen Hohlkörper mit zumindest einem oder mehreren Hohlräumen darstellt.

3. Gelenkimplantat nach einem der Patentansprüche 1 oder 2, **dadurch gekennzeichnet, dass** der stiftförmige Körper des Gelenkimplantats (1) eine zylindrische oder konische Form aufweist.

4. Gelenkimplantat nach einem der Patentansprüche 1 bis 3, **dadurch gekennzeichnet, dass** das Material des stiftförmigen Körpers
ein Polymer, insbesondere PA, PEK, PEKK, PEEK, UHMWPE oder PCL,
ein Metall, insbesondere Ti, Edelstahl, oder eine Metall-Legierung, insbesondere Ti64 oder CoCr,
eine Keramik, insbesondere Al2O3, ZrO2 oder Ca3(PO4)2, Si₃N₄, oder
Kombinationen von diesen Materialien aufweist.

5. Gelenkimplantat nach einem der Patentansprüche 1 bis 4, **dadurch gekennzeichnet, dass** zumindest der Deckenbereich (12) des Gelenkimplantats (1) eine Aussparung (16) zum Einbringen eines Drehmoments aufweist.

6. Gelenkimplantat nach Patentanspruch 5, **dadurch gekennzeichnet, dass** die Aussparung (16) die Form eines Schlitzes, Kreuzschlitzes, Innen-Mehrkant oder Torx aufweist.

7. Gelenkimplantat nach einem der Patentansprüche 1 bis 6, **dadurch gekennzeichnet, dass** die Gewindestruktur (15) des Gelenkimplantats (1) eine Gewindesteigung (GS) von 0,5 mm bis 1 mm aufweist.

8. Gelenkimplantat nach einem der Patentansprüche 1 bis 7, **dadurch gekennzeichnet, dass** der Bodenbereich (11) des Gelenkimplantats (1) eine Spitze aufweist und die Gewindestruktur (15) selbstformend, selbstprägend oder selbstschneidend ausgestaltet ist.

9. Gelenkimplantat nach einem der Patentansprüche 1 bis 7, **dadurch gekennzeichnet, dass** die hydrophobe Oberfläche durch eine hydrophobe chemische Beschichtung realisiert ist.

10. Gelenkimplantat nach Patentanspruch 9, **dadurch gekennzeichnet, dass** die hydrophobe chemische Beschichtung ein segmentiertes Polyurethan oder Polyelektrolyt oder ein hydrophob funktionalisiertes Chitosan oder Chitosan-Derivat aufweist.

11. Gelenkimplantat nach einem der Patentansprüche 1 bis 10, **dadurch gekennzeichnet, dass** die hydrophobe Oberfläche durch eine hydrophobe Nanostrukturierung eines Untergrunds realisiert ist.

12. Gelenkimplantat nach einem der Patentansprüche 1 bis 12, **dadurch gekennzeichnet, dass** der stiftförmige Körper des Gelenkimplantats (1) eine Länge (L) von 0,6 cm bis 2,2 cm, insbesondere von 1,25 cm, aufweist.

13. Gelenkimplantat nach einem der Patentansprüche 1 bis 12, **dadurch gekennzeichnet, dass** der stiftförmige Körper des Gelenkimplantats (1) einen Gewindedurchmesser (GD) von 2 mm bis 6 mm, insbesondere 3 mm, aufweist.

14. Gelenkimplantat nach einem der Patentansprüche 1 bis 13, **dadurch gekennzeichnet, dass** zumindest der Deckenbereich (12), insbesondere der gesamte stiftförmige Körper, des Gelenkimplantats (1) einen Wachstumsfaktor zur Begünstigung einer chondrozytären Differenzierung von mesenchymalen Stammzellen aufweist, insbesondere FGF-1, FGF-2, FGF-10 bis FGF-22, SDF-1, IGF-1, PDGF, TGF-β1 und TGF-β3, BMP-2 und BMP-7, OP-1, PRP oder bio-inertes Polyamid.

15. Gelenkimplantat nach einem der Patentansprüche 1 bis 14, **dadurch gekennzeichnet, dass** zumindest der Bodenbereich (11) des Gelenkimplantats (1) eine hydrophile Oberfläche zur Begünstigung einer osteoblastären Differenzierung von mesenchymalen Stammzellen aufweist.

## Claims

1. Joint implant for new tissue formation at a joint, wherein the joint implant (1) comprises a rod-shaped body with a base area (11), a cover area (12) and a sleeve area (13),
wherein a thread structure (15) is at least partially formed on the sleeve area (13) of the joint implant (1), and
**characterized in that** at least the cover area (12) and the thread structure (15), in particular the entire rod-shaped body, of the joint implant (1) have an artificial trabecular structure (14) produced by a 3D printing process such that a hydrophobic surface for facilitating chondrocyte differentiation of mesenchymal stem cells is implemented.

2. Joint implant according to Claim 1, **characterized in that** the rod-shaped body of the joint implant (1) is a hollow body with at least one or a plurality of hollow spaces.

3. Joint implant according to one of Claims 1 or 2, **characterized in that** the rod-shaped body of the joint implant (1) has a cylindrical or conical shape.

4. Joint implant according to one of Claims 1 to 3, **characterized in that** the material of the rod-shaped body comprises
a polymer, in particular PA, PEK, PEKK, PEEK, UHMWPE or PCL,
a metal, in particular Ti, stainless steel, or a metal alloy, in particular Ti64 or CoCr,
a ceramic, in particular Al2O3, ZrO2 or Ca3(PO4)2, Si₃N₄, or
combinations of these materials.

5. Joint implant according to one of Claims 1 to 4, **characterized in that** at least the cover area (12) of the joint implant (1) comprises a recess (16) for applying torque.

6. Joint implant according to Claim 5, **characterized in that** the recess (16) has the form of a slot, cross slot, inner polygon or Torx.

7. Joint implant according to one of Claims 1 to 6, **characterized in that** the thread structure (15) of the joint implant (1) has a thread pitch (GS) of 0.5 mm to 1 mm.

8. Joint implant according to one of Claims 1 to 7, **characterized in that** the base area (11) of the joint implant (1) has a point and the thread structure (15) has a self-forming, self-tapping or self-cutting configuration.

9. Joint implant according to one of Claims 1 to 7, **characterized in that** the hydrophobic surface is implemented by means of a hydrophobic chemical coating.

10. Joint implant according to Claim 9, **characterized in that** the hydrophobic chemical coating comprises a segmented polyurethane or polyelectrolyte or a hydrophobically functionalized chitosan or chitosan derivative.

11. Joint implant according to one of Claims 1 to 10, **characterized in that** the hydrophobic surface is implemented by hydrophobic nanostructuring of a substrate.

12. Joint implant according to one of Claims 1 to 12, **characterized in that** the rod-shaped body of the joint implant (1) has a length (L) of 0.6 cm to 2.2 cm, in particular 1.25 cm.

13. Joint implant according to one of Claims 1 to 12, **characterized in that** the rod-shaped body of the joint implant (1) has a thread diameter (GD) of 2 mm to 6 mm, in particular 3 mm.

14. Joint implant according to one of Claims 1 to 13, **characterized in that** at least the cover area (12), in particular the entire rod-shaped body, of the joint implant (1) comprises a growth factor for facilitating chondrocyte differentiation of mesenchymal stem cells, in particular FGF-1, FGF-2, FGF-10 to FGF-22, SDF-1, IGF-1, PDGF, TGF-β1 and TGF-β3, BMP-2 and BMP-7, OP-1, PRP or bioinert polyamide.

15. Joint implant according to one of Claims 1 to 14, **characterized in that** at least the base area (11) of the joint implant (1) has a hydrophilic surface for facilitating osteoblast differentiation of mesenchymal stem cells.

## Revendications

1. Implant articulaire pour la régénération tissulaire au niveau d'une articulation, l'implant articulaire (1) présentant un corps en forme de tige doté d'une région de base (11), d'une région supérieure (12) et d'une région d'enveloppe (13),
une structure filetée (15) étant au moins partiellement formée sur la région d'enveloppe (13) de l'implant articulaire (1), et
**caractérisé en ce qu'**au moins la région supérieure (12) et la structure filetée (15), en particulier tout le corps en forme de tige, de l'implant articulaire (1) présentent une structure trabéculaire artificielle (14), laquelle est fabriquée par un procédé d'impression 3D, de telle sorte qu'une surface hydrophobe soit réalisée pour favoriser une différenciation chondrocytaire de cellules souches mésenchymateuses.

2. Implant articulaire selon la revendication 1, **caractérisé en ce que** le corps en forme de tige de l'implant articulaire (1) constitue un corps creux doté d'au moins un ou plusieurs espaces creux.

3. Implant articulaire selon l'une des revendications 1 ou 2, **caractérisé en ce que** le corps en forme de tige de l'implant articulaire (1) présente une forme cylindrique ou conique.

4. Implant articulaire selon l'une des revendications 1 à 3, **caractérisé en ce que** le matériau du corps en forme de tige présente
un polymère, en particulier PA, PEK, PEKK, PEEK, UHMWPE ou PCL,
un métal, en particulier Ti, de l'acier fin, ou un alliage métallique, en particulier Ti64 ou CoCr,
une céramique, en particulier Al2O3, ZrO2 ou Ca3(PO4)2, Si₃N₄, ou
des combinaisons de ces matériaux.

5. Implant articulaire selon l'une des revendications 1 à 4, **caractérisé en ce qu'**au moins la région supérieure (12) de l'implant articulaire (1) présente un évidement (16) pour l'application d'un couple.

6. Implant articulaire selon la revendication 5, **caractérisé en ce que** l'évidement (16) présente la forme d'une fente, d'une fente cruciforme, d'une empreinte polygonale intérieure ou Torx.

7. Implant articulaire selon l'une des revendications 1 à 6, **caractérisé en ce que** la structure filetée (15) de l'implant articulaire (1) présente un pas de filetage (GS) de 0,5 mm à 1 mm.

8. Implant articulaire selon l'une des revendications 1 à 7, **caractérisé en ce que** la région de base (11) de l'implant articulaire (1) présente une pointe et la structure filetée (15) est configurée de manière autoformante, autotaraudante ou autocoupante.

9. Implant articulaire selon l'une des revendications 1 à 7, **caractérisé en ce que** la surface hydrophobe est réalisée par un revêtement chimique hydrophobe.

10. Implant articulaire selon la revendication 9, **caractérisé en ce que** le revêtement chimique hydrophobe présente un polyuréthane segmenté ou un polyélectrolyte ou un chitosane hydrophobe fonctionnalisé ou un dérivé de chitosane.

11. Implant articulaire selon l'une des revendications 1 à 10, **caractérisé en ce que** la surface hydrophobe est réalisée par une nanostructuration hydrophobe d'un substrat.

12. Implant articulaire selon l'une des revendications 1 à 12, **caractérisé en ce que** le corps en forme de tige de l'implant articulaire (1) présente une longueur (L) de 0,6 cm à 2,2 cm, en particulier de 1,25 cm.

13. Implant articulaire selon l'une des revendications 1 à 12, **caractérisé en ce que** le corps en forme de tige de l'implant articulaire (1) présente un diamètre de filetage (GD) de 2 mm à 6 mm, en particulier de 3 mm.

14. Implant articulaire selon l'une des revendications 1 à 13, **caractérisé en ce qu'**au moins la région supérieure (12), en particulier tout le corps en forme de tige, de l'implant articulaire (1) présente un facteur de croissance pour favoriser une différenciation chondrocytaire de cellules souches mésenchymateuses, en particulier FGF-1, FGF-2, FGF-10 à FGF-22, SDF-1, IGF-1, PDGF, TGF-βl et TGF-β3, BMP-2 et BMP-7, OP-1, PRP ou du polyamide bio-inerte.

15. Implant articulaire selon l'une des revendications 1 à 14, **caractérisé en ce qu'**au moins la région de base (11) de l'implant articulaire (1) présente une surface hydrophile pour favoriser une différenciation ostéoblastique de cellules souches mésenchymateuses.
